# FASCICULE DE BREVET EUROPEEN

(11) **EP 3 367 881 B1**
(45) Date de publication et mention de la délivrance du brevet: **30.09.2020**
(21) Numéro de dépôt: 16809472.0
(22) Date de dépôt: 28.10.2016
(51) Int. Cl.: A61B 3/12, A61B 3/15

(54) **DISPOSITIF DE MESURE DE LA VITESSE DU SANG DANS UN VAISSEAU SANGUIN DE L'OEIL**
VORRICHTUNG ZUR MESSUNG DER GESCHWINDIGKEIT VON BLUT IN EINEM BLUTGEFÄSS DES AUGES
DEVICE FOR MEASURING THE SPEED OF BLOOD IN A BLOOD VESSEL OF THE EYE

(30) Priorité: 30.10.2015 FR 1560450
(43) Date de publication de la demande: 05.09.2018
(73) Titulaire: Université Grenoble Alpes, 38400 Saint Martin d'Hères (FR)
(72) Inventeur: GEISER, Martial Henri, 1950 Sion (CH); TRUFFER, Frédéric, 3928 Randa (CH); STRESE, Helene, 32239 Peckelsheim (DE); CHIQUET, Christophe, 38330 Montbonnot-Saint-Martin (FR)
(74) Mandataire: Cabinet Chaillot
(86) Numéro de dépôt international: PCT/FR2016/052818
(87) Numéro de publication internationale: WO 2017/072468

(56) Documents cités:
- CN-A- 1 376 445
- US-A- 5 900 928
- US-A1- 2005 254 008
- US-A1- 2015 092 195

## Description

La présente demande de brevet revendique la priorité de la demande de brevet français FR15/60450 qui sera considérée comme faisant partie intégrante de la présente description.

### Domaine

La présente demande concerne un dispositif de mesure de la vitesse du sang dans un vaisseau sanguin de l'oeil. Ce dispositif est couramment appelé vélocimètre laser Doppler et désigné par le sigle LDV de l'anglais Laser Doppler Velocimeter.

### Exposé de l'art antérieur

Un vélocimètre laser Doppler est décrit dans le brevet des Etats-Unis d'Amérique N° 5900928 dont l'un des inventeurs est l'un des inventeurs de la présente demande.

La figure 1 ci-jointe reproduit le dessin de la figure 2 de ce brevet US 5900928, les références ayant été modifiées. Un laser 1 envoie à travers un diaphragme 3 un faisceau lumineux 5 vers un système optique 7 qui envoie ce faisceau 5 vers un oeil 10 et plus particulièrement vers un vaisseau sanguin 12 du fond de cet oeil. Le vaisseau rétrodiffuse de la lumière. Le système optique 7 ré-adresse, via un miroir 16, des pinceaux 14 et 15 de la lumière rétrodiffusée vers un diaphragme 17 d'entrée d'un système de détection 20 qui permet notamment de déterminer la vitesse du flux sanguin dans le vaisseau sanguin. Le système optique 7 est propre à assurer des liaisons confocales entre les différents plans du système.

Le dispositif décrit dans le brevet US 5900928 fonctionne de façon satisfaisante mais est très délicat à régler notamment en raison du couplage confocal des diverses pupilles d'entrée et de sortie. <Insérer la page 2A>

### Résumé

Ainsi, un objet de l'invention est de prévoir un système assurant les mêmes fonctions de vélocimétrie laser Doppler que celles décrites dans le brevet US 5900928 mais qui soit plus simple à réaliser et à régler.

Un mode de réalisation prévoit un dispositif de mesure de la vitesse du sang dans un vaisseau sanguin de l'oeil comprenant : une source lumineuse cohérente pour produire un faisceau source ; deux détecteurs munis de diaphragmes d'entrée pour recevoir deux pinceaux lumineux rétrodiffusés par un vaisseau sanguin ; un système optique comprenant un prisme de Dove traversé par le faisceau source et les pinceaux rétrodiffusés et adapté à être réglé en rotation autour d'un axe parallèle au faisceau source le traversant, et un système de focalisation adapté à faire l'image d'un vaisseau sanguin sur les diaphragmes d'entrée ; un processeur de signal adapté à traiter les signaux des détecteurs ; et un élément optique insérable pendant la phase de réglage du dispositif pour transformer le spot lumineux fourni par la source lumineuse en une ligne coplanaire avec le faisceau source et les pinceaux rétrodiffusés sélectionnés par les diaphragmes, le dispositif étant couplé à une caméra de fond de l'oeil pour viser dans le fond de l'oeil un vaisseau sanguin choisi et pour aligner ladite ligne sur la tangente au vaisseau sanguin au point d'éclairement au moyen d'une rotation du prisme de Dove.

Selon un mode de réalisation, la source lumineuse est un laser infrarouge.

La demande de brevet des Etats-Unis d'Amérique N° 2005/254008 décrit un dispositif de surveillance de flux sanguin dans la rétine à l'aide d'un ophtalmoscope laser à balayage linéaire.

Selon un mode de réalisation, l'élément optique de transformation d'une tache lumineuse en une ligne est un réseau ou une lentille cylindrique.

### Brève description des dessins

Ces caractéristiques et avantages, ainsi que d'autres, seront exposés en détail dans la description suivante de modes de réalisation particuliers faite à titre non limitatif en relation avec les figures jointes parmi lesquelles :

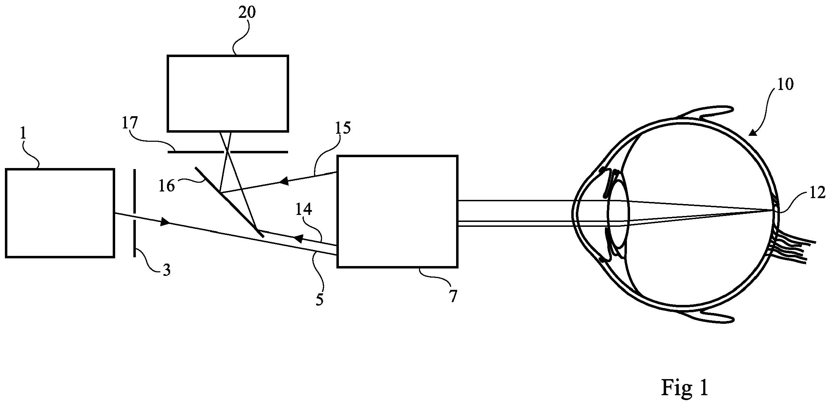

la figure 1, décrite précédemment, représente un vélocimètre laser Doppler tel que décrit dans le brevet US 59009028 ;
la figure 2 est un schéma de principe d'un mode de réalisation d'un vélocimètre laser Doppler ;
la figure 3 représente un détail du vélocimètre de la figure 2 ; et
la figure 4 représente un mode de réalisation du vélocimètre de la figure 2.

Par souci de clarté, de mêmes éléments ont été désignés par de mêmes références aux différentes figures.

### Description détaillée

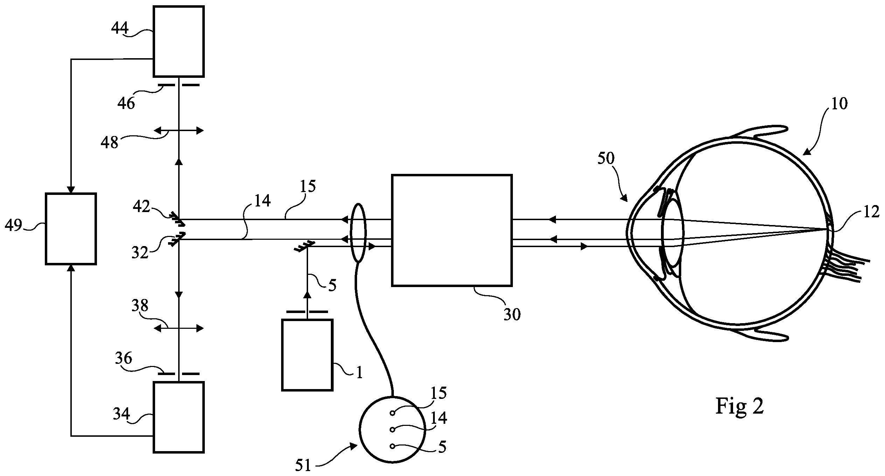

Comme le représente la figure 2, un mode de réalisation d'un vélocimètre destiné à l'analyse d'un vaisseau sanguin 12 d'un oeil 10, comprend une source laser 1 destinée à émettre un faisceau lumineux 5 vers le fond de l'oeil 10. Le faisceau laser passe par un système optique 30. La lumière rétrodiffusée par le vaisseau sanguin 12 repasse dans le système optique 30. Un pinceau 14 de cette lumière rétrodiffusée est renvoyé par un miroir 32 vers un détecteur 34 précédé d'un diaphragme 36 et d'une lentille de focalisation 38. Un autre pinceau 15 de cette lumière rétrodiffusée est renvoyé par un miroir 42 vers un détecteur 44 précédé d'un diaphragme 46 et d'une lentille de focalisation 48. Les détecteurs 34 et 44 sont couplés à un processeur de signal 49.

Une première fonction du système optique 30 est de conjuguer les ouvertures des diaphragmes 36 et 46 avec le vaisseau sanguin 12. La section 51 représente les points d'impact du faisceau émis 5 et des pinceaux de retour 14 et 15 dans un plan perpendiculaire au plan de la figure. Cette section montre que le système optique 30 est configuré et que les diaphragmes 36 et 46 sont disposés de manière que les pinceaux optiques 14 et 15 reçus par les détecteurs et le faisceau 5 émis par le laser sont conjugués de façon à définir un plan.

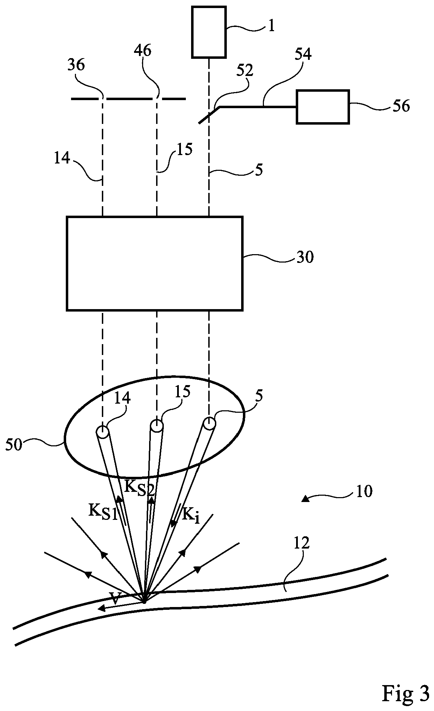

Le système optique 30 a une deuxième fonction qui est de permettre une rotation du plan du faisceau émis et des pinceaux reçus dans le plan de la pupille 50. Cette fonction est assurée par exemple par un prisme de Dove. On peut en conséquence, comme on le décrira plus en détails en relation avec la figure 3, faire tourner le plan défini ci-dessus autour de l'axe optique du système de façon à ce que ce plan contienne la tangente au vaisseau sanguin au point d'impact du faisceau laser sur ce vaisseau.

Alors la détection des deux pinceaux lumineux rétrodiffusés dans des directions différentes avec un angle connu entre ces directions permet de calculer la vitesse du sang dans le vaisseau sanguin alors même que l'angle d'incidence exact du faisceau laser n'est pas connu. Il convient de plus que le plan défini ci-dessus contienne la tangente au vaisseau sanguin au point d'éclairement de celui-ci.

La figure 3 représente une partie du dispositif décrit précédemment. On peut voir en haut de la figure le laser 1 émettant le faisceau 5, le diaphragme 36 recevant le pinceau lumineux 14, et le diaphragme 46 recevant le pinceau lumineux 15. Le faisceau 5 passe dans le système optique 30 et arrive au niveau de la pupille d'entrée 50 de l'oeil 10. Le faisceau 5 est dirigé selon un vecteur Ki vers un point d'un vaisseau sanguin 12 d'où sont rétrodiffusés des faisceaux dont on prélève les pinceaux 14 et 15. Le système est agencé comme indiqué précédemment pour que les vecteurs directeurs K_{S1} et K_{S2} des pinceaux 14 et 15 soient coplanaires avec le vecteur incident Ki.

D'autre part, comme on l'a indiqué précédemment, on souhaite que le vecteur tangent au vaisseau sanguin à l'endroit de l'impact (colinéaire avec le vecteur vitesse V du sang dans le vaisseau sanguin) soit également coplanaire avec les vecteurs Ki, K_{S1} et K_{S2}. La rotation du prisme de Dove inclus dans le système optique 30 permet d'assurer cette coplanarité.

Néanmoins, il se pose un problème pour vérifier expérimentalement que les quatre vecteurs V, Ki, KS1 et KS2 sont bien coplanaires.

Pour s'assurer de cette coplanarité, on utilise un élément optique amovible 52, par exemple un réseau optique ou une lentille cylindrique, monté au bout d'un bras basculant 54 relié au châssis 56 du dispositif. Ce réseau ou cette lentille cylindrique est choisi et orienté de sorte que la tache circulaire du faisceau laser est transformée en une ligne, cette ligne s'étendant dans le plan du faisceau 5 et des pinceaux 14 et 15 reçus par les diaphragmes. Alors, au niveau du fond de l'oeil, l'impact du faisceau 5 devient une ligne située dans le plan des vecteurs Ki, KS1 et KS2. La trace de cette ligne pourra être observée simultanément par une caméra de fond de l'oeil (un dispositif couramment utilisé par les opticiens et les ophtalmologues) ou directement par un observateur. On pourra ainsi régler angulairement le prisme de Dove pour que les quatre vecteurs V, Ki, KS1 et KS2 soient bien coplanaires. Si le laser 1 émet, comme cela est le cas courant, un faisceau dans le domaine de l'infrarouge, on utilisera une caméra sensible à l'infrarouge.

Bien entendu, la présente invention est susceptible de nombreuses variantes classiques en optique et notamment, par exemple par souci de compacité, divers miroirs de renvoi assurant les repliements de faisceaux peuvent être prévus.

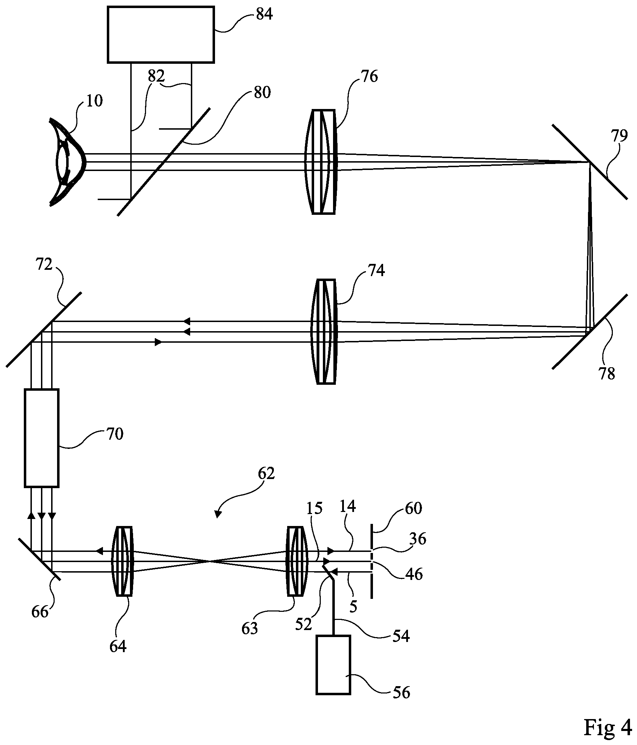

Un exemple de réalisation particulier est illustré en figure 4, dans lequel une plaque d'ouverture d'entrée 60 comprend les diaphragmes 36 et 46 susmentionnés ainsi qu'une ouverture pour laisser passer le faisceau laser 5. Un objectif 62 comprend des lentilles 63 et 64. La sortie de cet objectif est envoyée par l'intermédiaire d'un miroir de repliement 66 à un prisme de Dove 70. Le faisceau sortant du prisme de Dove 70 pourrait être envoyé directement vers l'oeil à analyser. Dans le mode de réalisation représenté, le faisceau est replié par un miroir 72 vers un système optique de grandissement inférieur à 1, comprenant deux lentilles 74 et 76 assurant la transmission des divers faisceaux vers l'oeil 10. Des miroirs de repliement de faisceau 78 et 79 sont prévus entre les lentilles 74 et 76. On obtient ainsi un dispositif de structure compacte. En outre, bien que cela n'ait pas été représenté, des moyens de rotation autour de son axe sont liés au prisme de Dove 70 pour régler la coplanarité des quatre vecteurs V, Ki, KS1 et KS2 comme cela a été mentionné en relation avec la figure 3.

On a également représenté en figure 4 le bras amovible 54 lié au châssis 56 et portant l'élément optique 52 permettant, pendant la phase de réglage de l'appareil, de transformer comme on l'a indiqué le spot lumineux fourni par le faisceau laser 5 en une ligne.

On a également représenté en figure 4, à titre d'exemple, une séparatrice 80 permettant de diriger les faisceaux d'entrée et de sortie 82 d'une caméra de fond de l'oeil 84 vers le fond de l'oeil. La caméra 84 permet de vérifier le positionnement du spot laser sur un vaisseau sanguin choisi. Elle permet aussi pendant une phase de réglage, d'orienter convenablement à l'aide du prisme de Dove la ligne fournie par l'élément optique 52 (par exemple un réseau ou une lentille cylindrique) sur le vaisseau sanguin visé. Comme on l'a noté, au lieu d'utiliser une caméra, un observateur pourra regarder directement le fond de l'oeil par l'intermédiaire de la séparatrice.

## Revendications

1. Dispositif de mesure de la vitesse du sang dans un vaisseau sanguin de l'oeil comprenant :
- une source lumineuse cohérente (1) pour produire un faisceau source (5) ;
- deux détecteurs (34, 44) munis de diaphragmes d'entrée (36, 46) pour recevoir deux pinceaux lumineux rétrodiffusés par un vaisseau sanguin ;
- un système optique (30) comprenant :
un prisme de Dove (70) traversé par le faisceau source et les pinceaux rétrodiffusés et adapté à être réglé en rotation autour d'un axe parallèle au faisceau source le traversant, et
un système de focalisation (62) adapté à faire l'image d'un vaisseau sanguin sur les diaphragmes d'entrée (36, 46) ;
- un processeur de signal (49) adapté à traiter les signaux des détecteurs ; et
- un élément optique amovible (52) insérable pendant la phase de réglage du dispositif pour transformer le spot lumineux fourni par la source lumineuse en une ligne coplanaire avec le faisceau source et les pinceaux rétrodiffusés sélectionnés par les diaphragmes,
le dispositif étant couplé à une caméra de fond de l'oeil pour viser dans le fond de l'oeil un vaisseau sanguin choisi et pour aligner ladite ligne sur la tangente au vaisseau sanguin au point d'éclairement au moyen d'une rotation du prisme de Dove.

2. Dispositif de mesure selon la revendication 1, dans lequel la source lumineuse est un laser infrarouge.

3. Dispositif selon la revendication 1, dans lequel l'élément optique (52) de transformation d'une tache lumineuse en une ligne est un réseau ou une lentille cylindrique.

## Patentansprüche

1. Vorrichtung zur Messung der Geschwindigkeit des Bluts in einem Blutgefäß des Auges, umfassend:
- eine kohärente Lichtquelle (1), um einen Quellstrahl (5) zu erzeugen;
- zwei Detektoren (34, 44), die mit Eingangsblenden (36, 46) ausgestattet sind, um zwei von einem Blutgefäß zurückgestreute Lichtbündel zu empfangen;
- ein optisches System (30), umfassend:
ein Dove-Prisma (70), das von dem Quellstrahl und den zurückgestreuten Bündeln durchquert wird und zum rotatorischen Einstellen um eine Achse, die parallel zu dem es durchquerenden Quellstrahl ist, geeignet ist, und
ein Fokussiersystem (62), das zum Erzeugen des Bildes eines Blutgefäßes auf den Eingangsblenden (36, 46) geeignet ist;
- einen Signalprozessor (49), der zum Verarbeiten der Signale der Detektoren geeignet ist; und
- ein lösbares optisches Element (52), das während der Einstellphase der Vorrichtung einsetzbar ist, um den von der Lichtquelle bereitgestellten Lichtspot in eine mit dem Quellstrahl und den zurückgestreuten, von den Blenden ausgewählten Bündeln koplanare Linie umzuwandeln,
wobei die Vorrichtung mit einer Augenhintergrundkamera gekoppelt ist, um im Augenhintergrund auf ein ausgewähltes Blutgefäß zu zielen und um die Linie auf der Tangente zum Blutgefäß auf den Beleuchtungspunkt mittels einer Rotation des Dove-Prismas auszurichten.

2. Messvorrichtung nach Anspruch 1, wobei die Lichtquelle ein Infrarotlaser ist.

3. Vorrichtung nach Anspruch 1, wobei das optische Element (52) zur Umwandlung eines Lichtflecks in eine Linie ein Netzwerk oder eine zylindrische Linse ist.

## Claims

1. A device for measuring the speed of blood in a blood vessel of the eye, comprising:
- a coherent light source (1) for generating a source beam (5);
- two detectors (34, 44) provided with entrance apertures (36, 46) to receive two light pencil beams backscattered by a blood vessel;
- an optical system (30) comprising;
a Dove prism (70) crossed by the source beam and the backscattered pencil beams and capable of being rotatably adjusted around an axis parallel to the source beam crossing it, and
a focusing system (62) capable of forming the image of a blood vessel on the entrance apertures (36, 46);
- a signal processor (49) capable of processing the signals from the detectors; and
- a removable optical element (52) which can be inserted during the device adjustment phase to transform the light spot supplied by the light source into a line coplanar with the source beam and the backscattered pencil beams selected by the apertures,
the device being coupled with a fundus camera to aim, into the fundus of the eye, a selected blood vessel and to align said line with the tangent to the blood vessel at the illumination point by means of a rotation of the Dove prism.

2. The measurement device of claim 1, wherein the light source is an infrared laser.

3. The device of claim 1, wherein the optical element (52) for transforming a light spot into a line is a grating or a cylindrical lens.
